## Europäisches Patentamt

## European Patent Office

## Office européen des brevets

(11) Veröffentlichungsnummer: **0 134 406**
**B1**

(12) **EUROPÄISCHE PATENTSCHRIFT**

(45) Veröffentlichungstag der Patentschrift:
09.03.88

(21) Anmeldenummer: **84105651.8**

(22) Anmeldetag: **18.05.84**

(51) Int. Cl.⁴: **A 61 B 17/58, A 61 F 2/30**

(54) Zuganker für Röhrenknochen.

(30) Priorität: **19.08.83 DE 3330062**

(43) Veröffentlichungstag der Anmeldung:
**20.03.85 Patentblatt 85/12**

(45) Bekanntmachung des Hinweises auf die Patenterteilung:
**09.03.88 Patentblatt 88/10**

(84) Benannte Vertragsstaaten:
**CH DE FR GB LI**

(56) Entgegenhaltungen:
**CH - A - 453 570**
**DE - B - 745 873**
**US - A - 2 077 804**

(73) Patentinhaber: **Waldemar Link GmbH & Co,
Barkhausenweg 10, D-2000 Hamburg 63 (DE)**

(72) Erfinder: **Keller, Arnold, An der Naherfurth 5,
D-2061 Kayhude (DE)**

(74) Vertreter: **Glawe, Delfs, Moll & Partner Patentanwälte,
Postfach 26 01 62 Liebherrstrasse 20,
D-8000 München 26 (DE)**

## Beschreibung

Die Erfindung bezieht sich auf eine Vorrichtung zum Verankern eines Zugelements in einem Röhrenknochen gemäss Oberbegriff des Anspruchs 1.

In manchen Fällen ist es notwendig oder vorteilhaft, in einem röhrenförmigen Knochen in Längsrichtung Druckspannungen aufzubauen, beispielsweise bei der Frakturbehandlung oder in Verbindung mit Endoprothesen, insbesondere bei Hüftgelenk-Endoprothesen.

Bekannt sind Vorrichtungen dieser Art, bei denen das Zugelement in der Tiefe des Röhrenknochens durch einen von aussen quer in den Knochen einzubringenden Anker befestigt werden muss. Der zusätzliche Eingriff zur Einbringung des Ankers von seitlich ausserhalb des Knochens her ist jedoch problematisch.

Aus der DE-C 745 873 ist eine Innenschiene für Röhrenknochen bekannt, die einen oder mehrere Anker zur Fixierung der Schiene in dem Knochen aufweist. Jeder Anker besteht aus einer gelenkig in der Schiene gelagerten Klinke, deren freies Ende über den Querschnitt der Innenschiene heraustritt und an deren anderem Ende ein Zugglied angreift, mit dessen Hilfe die Klinke so weit um die Achse verdreht werden kann, bis ihr freies Ende an der Corticalis des Röhrenknochens verkrallt. Bei der ähnlichen Anordnung nach der CH-A 453 570 sind am Ende eines im Inneren der Innenschiene gelegenen Zugglieds Anker gelenkig befestigt, deren Enden in der Art von Widerhaken durch Schlitze in der Innenschiene herausragen und sich unter der Kraft des Zugglieds gegen die Corticalis des Röhrenknochens verkeilen. Der Nachteil solcher Vorrichtungen ist die für die Spreizung der Dornen notwendige Mechanik, die zumindest ein Zugglied und ein Druckglied (Innenschiene) benötigt.

Der Erfindung liegt die Aufgabe zugrunde, eine Vorrichtung der eingangs genannten Art zu schaffen, die diese Nachteile vermeidet. Die Lösung besteht in den kennzeichnenden Merkmalen des Anspruchs 1, zweckmässigerweise in Verbindung mit den Merkmalen eines oder mehrerer Unteransprüche.

Beim Einführen der Zugstange klappt der Anker dank seiner gelenkigen Verbindung mit der Zugstange mehr oder weniger an diese heran, so dass die Querabmessungen der aus Zugstange und Anker sich zusammensetzenden Vorrichtung hinreichend kleiner sind als der Durchmesser der Markhöhle, in die sie einzuschieben ist. Bei Zugbelastung der Zugstange klappt die Platte durch den einseitigen Kraftangriff von der Zugstange weg und verklemmt sich an den Wänden der Markhöhle. Dieses Verklemmen kann durch eine an den Enden der Platte oder ggf. auch seitlich daran angebrachte Verzahnung gefördert werden.

Der Anker hält solange stabil in seiner Verankerungsstellung, solange sein Winkel gegenüber der Wandung der Markhöhle steiler ist als der Reibungswinkel. Da der Reibungskoeffizient des scharfkantigen Ankers gegenüber der rauhen Corticalis sehr hoch ist, erlaubt diese Regel dem Anker einen sehr grossen Winkelspielraum. Daraus folgen auch sehr weite Grenzen für seine Längenbemessung. Eine genaue Abstimmung auf den Durchmesser der Markhöhle ist daher nicht erforderlich.

Die Exzentrizität der Verbindung zwischen Anker und Zugstange braucht nur so gross zu sein, dass sich die Verkantung des Ankers in der Markhöhle und damit die gewünschte Verklemmung zwischen den Markhöhlenwänden sicher ergibt. Sie soll auch nicht wesentlich grösser sein als es für diesen Zweck erforderlich ist, damit der dadurch bewirkte Unterschied in der Belastung der beiden Ankerenden möglichst gering ist. In diesem Sinne ist auch das Anspruchsmerkmal aufzufassen, dass die gelenkige Verbindung zwischen Anker und Zugstange an einem Ende des Ankers vorgesehen ist.

Die Schwenkachse des Gelenks verläuft quer zu den Längsrichtungen der Zugstange und des Ankers. Eine sehr einfache Ausführung des Gelenks besteht darin, dass die Zugstange durch eine Bohrung im Anker geführt ist, deren Durchmesser wesentlich grösser ist als derjenige der Zugstange, wobei die Zugstange vor und hinter der Bohrung jeweils eine Verdickung, beispielsweise in Kugelform aufweist, wie sie durch eine Schweissperle erzeugt werden kann.

Die Erfindung wird im folgenden näher unter Bezugnahme auf die Zeichnung erläutert, die ein vorteilhaftes Ausführungsbeispiel veranschaulicht. Darin zeigen:

Fig. 1 eine Seitenansicht der Vorrichtung,
Fig. 2 eine Endansicht der Vorrichtung,
Fig. 3 das Prinzip der Verankerung bei Frakturbehandlung und
Fig. 4 die Verwendung der Vorrichtung im Zusammenhang mit einer Hüftgelenk-Endoprothese.

Die Zugstange 1 weist am einen Ende zwei kugelförmige Verdichtungen 2, 3 auf. Dazwischen durchquert sie die Bohrung 4 des plättchenförmigen Ankers 5, wobei der Bohrungsdurchmesser geringer ist als der Durchmesser der kugelförmigen Verdickungen 2 und 3. Der Anker 5 hat ovalen Umriss, wobei seine kürzere Querachse kürzer ist als der Durchmesser des Markkanals, während seine längere Achse – vorzugsweise um den Faktor 1,05–1,4 – grösser ist als der Markhöhlendurchmesser. Die Enden weisen eine Zahnung 6 zur besseren Verankerung an der Innenfläche der Corticalis des Knochens auf.

Der Durchmesser der Bohrung 4 ist so viel grösser als der Stangendurchmesser, dass der Anker an der Stange in eine Lage kippen kann, in der seine quer zur Stangenlängsrichtung gemessene Abmessung kleiner ist als der Markhöhlendurchmesser.

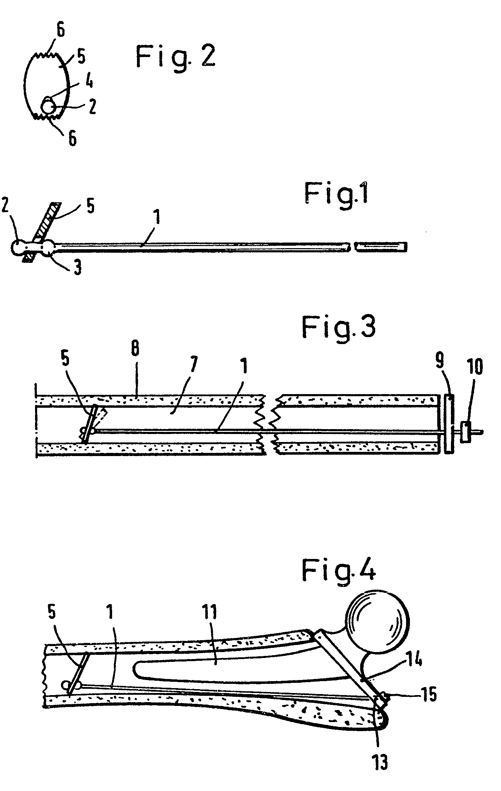

Dies letztere Merkmal gestattet es, die Vorrichtung gemäss Fig. 3 in Längsrichtung in die Markhöhle 7 eines Knochens 8 einzuschieben, wobei der Anker gegenüber der Zugstange in die strichpunktiert dargestellte Lage geschwenkt ist. Wird danach Zug auf die Zugstange ausgeübt, so kippt der Anker in die mit durchgezogenen Linien veranschaulichte Lage, in der er sich an den Innenwänden der Corticalis 8 festklemmt und dadurch ein zuverlässiges Gegenlager bildet, wenn die getrennt dargestellten Knochenteile in Längsrichtung mittels der Zugstange, die am anderen Ende mit einer Gegenlagerplatte 9 und einer Mutter 10 versehen ist, zusammengespannt werden.

Fig. 4 zeigt eine analoge Anwendung im Zusammenhang mit einer Hüftgelenk-Oberschenkelprothese 11. Die mittels des Ankers 5 im Oberschenkelknochen 12 verankerte Zugstange 1 ist proximal durch eine Bohrung 13 in der Halsauflageplatte 14 der Prothese 11 geführt und mittels einer Mutter 15 verspannt. Der mediale Teil des Knochens 12 kann dadurch stärker an der Kraftübertragung auf die Prothese beteiligt werden.

**Patentansprüche**

1. Zuganker zur Verankerung in Röhrenknochen, bestehend aus einem Zugglied und einem im Knochen zu befestigenden, mit dem Zugglied verbundenen, länglich ausgebildeten Anker, der nahe seinem einen Ende gelenkig mit dem Zugglied derart verbunden ist, dass er in der Knochenmarkhöhle bei Zugbelastung des Zugglieds in eine Lage kippen kann, in der er sich an den Wänden der Markhöhle verklemmt, dadurch gekennzeichnet, dass der Anker so ausgestaltet ist, dass er sich in der genannten Lage an seinen beiden Enden gegen die Wände der Markhöhle stützen kann.

2. Vorrichtung nach Anspruch 1, dadurch gekennzeichnet, dass der Anker plättchenförmig ausgebildet ist und zumindest an seinen Enden bogenförmig begrenzt ist.

3. Vorrichtung nach Anspruch 2, dadurch gekennzeichnet, dass der Anker oval begrenzt ist.

4. Vorrichtung nach einem der Ansprüche 1 bis 3, dadurch gekennzeichnet, dass die Enden des Ankers gezahnt sind.

**Claims**

1. A tension rod to be anchored in a long bone, comprising a tension member and an anchor of elongate shape, connected to the tension member and to be secured in the bone, which anchor near to its end is articulately connected to the tension member so that it can tilt in the medullary cavity under tensile loading of the tension member into a position in which it jams against the walls of the medullary cavity, characterised in that the anchor is so designed that in the said position it can bear at both ends against the walls of the medullary cavity.

2. A device according to claim 1, characterised in that the anchor is of lamellar form and is bounded arcuately at least at its ends.

3. A device according to Claim 2, characterised in that the anchor is bounded ovally.

4. A device according to any of claims 1 to 3, characterised in that the ends of the anchor are toothed.

**Revendications**

1. Tirant d'ancrage pour l'ancrage dans des os longs, se composant d'un membre tendu (1) et d'un élément d'ancrage (5) réalisé sous forme oblongue, raccordé au membre tendu (1) et destiné à être fixé dans l'os, élément d'ancrage qui s'articule, à proximité de l'une de ses extrémités, sur le membre tendu (1), de telle manière que sous l'effet de la contrainte de traction exercée sur le membre tendu, il puisse basculer dans le canal médullaire de l'os dans une position dans laquelle il se coince sur les parois du canal médullaire, caractérisé en ce que l'élément d'ancrage (5) est réalisé de telle manière que, dans ladite position, il puisse prendre appui par ses deux extrémités sur les parois du canal médullaire (7).

2. Dispositif selon la revendication 1, caractérisé en ce que l'élément d'ancrage (1) est réalisé sous forme de plaquette et présente, au moins à ses extrémités, un pourtour en arc de cercle.

3. Dispositif selon la revendication 2, caractérisé en ce que l'élément d'ancrage (1) présente un pourtour ovale.

4. Dispositif selon l'une quelconque des revendications 1 à 3, caractérisé en ce que les extrémités de l'élément d'ancrage (1) sont dentelées.

0 134 406

Fig. 2

Fig. 1

Fig. 3

Fig. 4

5